# FASCICULE DE BREVET EUROPEEN

(11) **EP 0 674 512 B1**
(45) Date de publication et mention de la délivrance du brevet: **18.03.1998**
(21) Numéro de dépôt: 94902019.2
(22) Date de dépôt: 10.12.1993
(51) Int. Cl.: A61K 31/425

(54) **APPLICATION DU RILUZOLE DANS LE TRAITEMENT DE LESIONS NEUROLOGIQUES LIEES A DES TRAUMATISMES**
VERWENDUNG VON RILUZOLE ZUR BEHANDLUNG VON TRAUMATISCHEN NEUROLOGISCHEN-SCHÄDIGUNGEN
APPLICATION OF RILUZOLE IN THE TREATMENT OF NEUROLOGICAL LESIONS RELATED TO TRAUMATIC INJURIES

(30) Priorité: 16.12.1992 FR 9215148
(43) Date de publication de la demande: 04.10.1995
(73) Titulaire: RHONE-POULENC RORER S.A., 92160 Antony (FR)
(72) Inventeur: DOBLE, Adam, F-75005 Paris (FR); LOUVEL, Erik, F-75010 Paris (FR); PRATT, Jérémy, F-94220 Charenton-le-Pont (FR); STUTZMANN, Jean-Marie, F-94440 Villecresnes (FR)
(74) Mandataire: Savina, Jacques
(86) Numéro de dépôt international: FR9301229
(87) Numéro de publication internationale: WO9413288

(56) Documents cités:
- EP-A- 0 050 551
- EP-A- 0 282 971
- NEUROSCIENCE LETTERS vol. 140, no. 2 , 22 Juin 1992 pages 225 - 230 J. PRATT ET AL. 'NEUROPROTECTIVE ACTIONS OF RILUZOLE IN RODENT MODELS OF GLOBAL AND FOCAL CEREBRAL ISCHAEMIA'
- THE JOURNAL OF NEUROSCIENCE vol. 9, no. 11 , 1989 pages 3720 - 3727 C. MALGOURIS ET AL. 'RILUZOLE, A NOVEL ANTIGLUTAMATE, PREVENTS MEMORY LOSS AND HIPPOCAMPAL NEURONAL DAMAGE IN ISCHAEMIC GERBILS'
- SOC. NEUROSC. ABSTR. vol. 14 , 1988 page 774 V.O. GARDNER ET AL. 'EXCITOTOXIC MEDIATED SPINAL CORD DAMAGE: POSSIBLE ROLE OF THE NMDA RECEPTOR'
- ACTA NEUROCHIRURGICA vol. 55, no. SUP , 1992 pages 49 - 55 R. BULLOCK ET AL. 'PREVENTION OF POST-TRAUMATIC EXCITOTOXIC BRAIN DAMAGE WITH NMDA ANTAGONIST DRUGS: A NEW STRATEGY FOR THE NINETIES'
- EUROPEAN JOURNAL OF PHARMACOLOGY vol. 175, no. 2 , 1990 pages 165 - 174 A.I. FADEN ET AL. 'EFFECTS OF COMPETITIVE AND NON-COMPETITIVE NMDA RECEPTOR ANTAGONISTS IN SPINAL CORD INJURY'
- SCIENCE vol. 244, no. 4906 , 1989 pages 798 - 800 A.I. FADEN ET AL. 'THE ROLE OF EXCITATORY AMINO ACIDS AND NMDA RECEPTORS IN TRAUMATIC BRAIN INJURY'
- NEUROPHARMACOLOGY vol. 24, no. 11 , 1985 pages 1085 - 1092 J. BENAVIDES ET AL. '2-AMINO-6-TRIFLUOROMETHOXY BENZOTHIAZOLE, A POSSIBLE ANTAGONIST OF EXCITATORY AMINO ACID NEUROTRANSMISSION-II'
- NEUROSCIENCE LETTERS vol. 147, no. 2 , 7 Décembre 1992 pages 209 - 212 A. CHERAMY ET AL. 'RILUZOLE INHIBITS THE RELEASE OF GLUTAMATE IN THE CAUDATE NUCLEUS OF THE CAT IN VIVO'

## Description

La présente invention concerne une nouvelle application thérapeutique du riluzole ou les sels pharmaceutiquement acceptables de ce composé.

Il est connu que le riluzole est utile comme médicament anticonvulsivant, anxiolytique et hypnotique (brevet EP 50551), dans le traitement de la schizophrénie (EP 305276), dans le traitement des troubles du sommeil et de la dépression (EP 305277), dans le traitement des désordres cérébrovasculaires et comme anesthésique (EP 282971).

Il a maintenant été trouvé de manière surprenante que ce composé peut aussi être utilisé dans le traitement des lésions neurologiques liées à des traumatismes et, en particulier, à des traumatismes spinaux, crâniens ou crânio-spinaux.

L'activité de ce produit a été mise en évidence sur les lésions de la moelle épinière dans le modèle de lésion de moelle chez le rat par la technique de compression au moyen d'un ballonnet au niveau médullaire lombaire décrite par ZILELI et coll, Acta Neurochi., 108, 140-147 (1991) et POINTILLART et coll, J. of Neurotrauma, 10, 2, 201-213 (1993). Le riluzole (4 mg/kg i.v./jour) est injecté 5 minutes après le traumatisme puis tous les jours pendant 7 jours. Dans le groupe non traité (10 rats), 2 rats sont morts au troisième jour, 7 rats n'ont pas récupéré de leur lésion, 1 rat a récupéré de sa lésion au septième jour. Dans le groupe traité au riluzole (10 rats), 1 rat est mort au ciquième jour, 1 rat n'a pas récupéré de sa lésion et 8 rats ont retrouvé leur motilité et leur potentiel évoqué somesthésique (SEP) entre le troisième et le septième jour.

L'activité de ce produit sur les lésions de la moelle épinière a également été mise en évidence soit dans le modèle de lésion de moelle chez le rat par la technique de compression ventrale décrite par E. C. BENZEL et coll., Journal of Spinal Disorders, 3, 4, 334-338 (1990), soit dans le modèle de tétraplégie traumatique induite par la compression de la moelle épinière par un microballonnet gonflable selon la technique décrite par D. MARTIN et coll, Journal of Neuroscience Research, 32, 539-550 (1992).

Dans ces tests, le riluzole diminue le déficit neurologique des animaux (paraplégie) lié à la lésion de la moelle épinière ainsi que les lésions histopathologiques (nécrose de la moelle). Cette diminution est généralement égale ou supérieure à 5%.

L'activité de ce produit est également démontrée dans le modèle de lésions médullaires sur 15 lapins "Fauve de Bourgogne" de 4 kg (± 200g). Les lapins sont divisés en trois groupes pour recevoir des traumatismes d'importance variable selon le protocole suivant :
a) préparation de l'animal : on injecte aux lapins intramusculairement 5 mg de valium® et 1/16 mg d'atropine. 30 minutes après, on met en place une perfusion salée isotonique et anesthésie les lapins par injection intraveineuse lente de 40 mg/kg de Nesdonal®. On met ensuite en place un cardioscope car l'animal peut présenter, surtout lors de réinjections de Nesdonal®, une apnée durable avec bradycardie.
b) enregistrement des potentiels évoqués somesthésiques (PES) : ces enregistrements précisent l'intégrité des voies sensitives de la moelle. La stimulation est réalisée au niveau du nerf sciatique poplité interne (SPI). L'intensité de stimulation est calculée pour évoquer un potentiel dans les fibres sensitives de gros calibre, ce qui est réalisé au niveau du seuil de stimulation motrice (mouvement minimal de la patte). Le recueil se fait au moyen d'une électrode plantée dans le scalp au niveau du cortex pariétal controlatéral. Une électrode de référence est placée sur la ligne médiane du scalp au niveau frontal (Fz). Un PES est enregistré avant la pose de la sonde pour servir de référence.
c) réalisation du traumatisme : le traumatisme est réalisé par gonflage du ballonnet d'une sonde de Fogarty French 3 placé dans le canal rachidien en position extradurale. Pour cela, on réalise une laminectomie lombaire basse. L'ouverture du ligament jaune permet le passage de la sonde jusqu'au niveau de la première vertèbre lombaire et la plaie opératoire est refermée. Un nouveau PES est enregistré pour s'assurer de l'absence de lésion fonctionnelle lors du passage de la sonde. La lésion est ensuite réalisée par gonflage du ballonnet avec des quantités variables d'air (0,2; 0,4 et 0,55 ml d'air) puis la sonde est retirée. Un nouveau PES est réalisé juste après le traumatisme et est comparé (amplitudes et latences) au PES de référence.
d) Les produits sont injectés par voie intrapéritonéale 1 fois par jour pendant 5 jours, à des doses comprises entre 1 et 8 mg/kg.
e) histologie : on prélève un bloc rachis-moelle comprenant le niveau lésé qui est mis dans du formol à 10%. Une semaine plus tard, la moelle est extraite du bloc (ce délai de fixation semble nécessaire pour éviter une lésion post-mortem). Une zone hémorragique visible à l'oeil nu montre le niveau du traumatisme. Des coupes histologiques étagées précisent l'extension des lésions.
f) résultats : le riluzole permet de diminuer le déficit neurologique lié à la lésion de la moelle épinière, de protéger les voies neurologiques sensitives et de diminuer la zone nécrotique hémorragique au sein de la substance grise de la moelle épinière. Ces diminutions sont généralement égales ou supérieures à 5%.

L'activité de ce produit dans les traumatismes crâniens a aussi été mise en évidence chez le rat selon la technique décrite par T. K. McINTOSH et col., Central Nervous System Trauma, 4, 2, 119-134 (1987).

Dans ce test, le riluzole améliore le score neurologique des animaux ayant subi un traumatisme crânien et réduit les lésions nécrotiques. Cette diminution est généralement égale ou supérieure à 5%.

Comme sels pharmaceutiquement acceptables peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments sont constitués par au moins le riluzole sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale ou parentérale.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les doses dépendent de l'effet recherché, de la durée du traitement et de la voie d'administration utilisée; elles sont généralement comprises entre 50 et 800 mg par jour par voie orale pour un adulte avec des doses unitaires allant de 25 à 200 mg de substance active et entre 25 et 600 mg par jour par voie intraveineuse pour un adulte avec des doses unitaires allant de 12,5 à 200 mg de substance active.

D'une façon générale, le médecin déterminera la posologie appropriée en fonction de l'âge, du poids et de tous les autres facteurs propres au sujet à traiter.

Les exemples suivants illustrent des médicaments selon l'invention :

### Exemple A

On prépare, selon la technique habituelle, des comprimés dosés à 50 mg de produit actif ayant la composition suivante :

| | |
|---|---|
| - Riluzole | 50 mg |
| - Mannitol | 64 mg |
| - Cellulose microcristalline | 50 mg |
| - Polyvidone excipient | 12 mg |
| - Carboxyméthylamidon sodique | 16 mg |
| - Talc | 4 mg |
| - Stéarate de magnésium | 2 mg |
| - Silice colloïdale anhydre | 2 mg |
| - Mélange de méthylhydroxypropylcellulose, polyéthylèneglycol 6000, dioxyde de titane (72-3,5-24,5) q.s.p. 1 comprimé pelliculé terminé à 245 mg | |

### Exemple B

On prépare, selon la technique habituelle, des gélules dosées à 50 mg de produit actif ayant la composition suivante :

| | |
|---|---|
| - Riluzole | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

### Exemple C

On prépare une solution injectable contenant 10 mg de produit actif ayant la composition suivante :

| | |
|---|---|
| - Riluzole | 10 mg |
| - Acide benzoïque | 80 mg |
| - Alcool benzylique | 0,06 cm3 |
| - Benzoate de sodium | 80 mg |
| - Ethanol à 95 % | 0,4 cm3 |
| - Hydroxyde de sodium | 24 mg |
| - Propylène glycol | 1,6 cm3 |
| - Eau q.s.p. | 4 cm3 |

L'invention concerne également le procédé de préparation de médicaments utiles dans le traitement des lésions neurologiques liées à des traumatismes et, en particulier, à des traumatismes spinaux, crâniens ou crânio-spinaux consistant à mélanger le riluzole ou les sels pharmaceutiquement acceptables de ce composé avec un ou plusieurs diluants et/ou adjuvants compatibles et pharmaceutiquement acceptables.

L'invention concerne également l'utilisation du riluzole ou de ses sels pharmaceutiquement acceptables pour la préparation d'un médicament pour le traitement d'un mammifère et, notamment l'homme, présentant des lésions neurologiques liées à des traumatismes et, en particulier, à des traumatismes spinaux, crâniens ou crânio-spinaux.

## Revendications

1. Application du riluzole ou les sels pharmaceutiquement acceptables de ce composé à la préparation de médicaments destinés au traitement des lésions neurologiques liées à des traumatismes.

2. Application selon la revendication 1 à la préparation de médicaments destinés au traitement des lésions neurologiques liées aux traumatismes spinaux.

3. Application selon la revendication 1 à la préparation de médicaments destinés au traitement des lésions neurologiques liées aux traumatismes crâniens.

4. Application selon la revendication 1 à la préparation de médicaments destinés au traitement des lésions neurologiques liées aux traumatismes cranio-spinaux.

5. Application selon l'une des revendications 1 à 4 pour obtenir un médicament utile par voie orale comprenant 25 à 200 mg de riluzole.

6. Application selon l'une des revendications 1 à 4 pour obtenir un médicament utile par voie intraveineuse comprenant 12,5 à 200 mg de riluzole.

## Claims

1. Use of riluzole or the pharmaceutically acceptable salts of this compound for the manufacture of medicaments intended for the treatment of neurological lesions associated with trauma.

2. Use according to claim 1, for the manufacture of medicaments intended for the treatment of neurological lesions associated with spinal trauma.

3. Use according to claim 1, for the manufacture of medicaments intended for the treatment of neurological lesions associated with cranial trauma.

4. Use according to claim 1, for the manufacture of medicaments intended for the treatment of neurological lesions associated with craniospinal trauma.

5. Use according to one of claims 1 to 4, for obtaining a medicament which can be used via the oral route comprising 25 to 200 mg of riluzole.

6. Use according to one of claims 1 to 4, for obtaining a medicament which can be used via the intravenous route comprising 12.5 to 200 mg of riluzole.

## Patentansprüche

1. Verwendung von Riluzol oder den pharmazeutisch akzeptablen Salzen dieser Verbindung zur Herstellung von Arzneimitteln für die Behandlung von neurologischen Schäden, die mit Traumata verbunden sind.

2. Verwendung nach Anspruch 1 zur Herstellung von Arzneimitteln für die Behandlung von neurologischen Schäden, die mit spinalen Traumata verbunden sind.

3. Verwendung nach Anspruch 1 zur Herstellung von Arzneimitteln für die Behandlung von neurologischen Schäden, die mit cranialen Traumata verbunden sind.

4. Verwendung nach Anspruch 1 zur Herstellung von Arzneimitteln für die Behandlung von neurologischen Schäden, die mit craniospinalen Traumata verbunden sind.

5. Verwendung nach einem der Ansprüche 1 bis 4 zur Herstellung eines auf oralem Wege anwendbaren Arzneimittels, das 25 mg bis 200 mg Riluzol umfaßt.

6. Verwendung nach einem der Ansprüche 1 bis 4 zur Herstellung eines auf intravenösem Wege anwendbaren Arzneimittels, das 12,5 mg bis 200 mg Riluzol umfaßt.
